# EUROPEAN PATENT SPECIFICATION

(11) **EP 0 533 434 B1**
(45) Date of publication and mention of the grant of the patent: **09.06.1999**
(21) Application number: 92308385.1
(22) Date of filing: 15.09.1992
(51) Int. Cl.: A61K 6/083

(54) **Aesthetic, opalescent cold-polymerizable dental materials**
Ästetische, opalisierende kaltpolymerisierbare Dentalmassen
Matériaux dentaires estéthiques, opalescents, polymérisables à froid

(30) Priority: 20.09.1991 US 763133
(43) Date of publication of application: 24.03.1993
(73) Proprietor: MINNESOTA MINING AND MANUFACTURING COMPANY, St. Paul, Minnesota 55133-3427 (US)
(72) Inventor: Holmes, Brian N., c/o Minnesota Mining and, St. Paul, Minnesota 55133-3427 (US); Bryan, Thomas T., c/o Minnesota Mining and, St. Paul, Minnesota 55133-3427 (US)
(74) Representative: Baillie, Iain Cameron

(56) References cited:
- EP-A- 0 270 472
- WO-A-90/08799
- US-A- 4 141 144
- US-A- 4 281 991

## Description

A variety of materials have been used to reinforce or to pigment cold-polymerizable (i.e., polymerizable without the use of elevated temperatures) dental composites, restoratives and cements. For example, U.S. Patent Nos. 4,281,991 and 4,029,632 describe dental prosthetic devices containing submicron silica (and in the case of the '991 patent, submicron aluminum oxide). The articles of the '991 patent are said to have opalescence.

### OTHER ART

For brevity, titanium dioxide with a particle diameter less than 0.2 micrometers will be referred to in this application as "microfine titania". Degussa Technical Bulletin No. 56 entitled "Aluminum Oxide C, Titanium Dioxide P 25-Two Highly Dispersed Metal Oxides from Degussa Produced by the AEROSIL®-Process" refers at page 8 to various uses for the microfine titania "Titanium Dioxide P 25", and states:
"In opposition to other pigments which are also used as stabilizers, Titanium Dioxide P 25 offers the advantage of being almost colorless and thus of having practically no effect on the shades of color specified.
"Because of its more opalescent than pigmenting effects, and because of the extreme fineness of its particles, Titanium Dioxide P 25 is used in cosmetics for the tinting of pigments, used in nail polishes and to improve the suspension properties of these pigments. Amounts of Titanium Dioxide P 25 varying from 0.5-1.0% are added and should be dispersed with the pigments in the polish. Ointments and creams can also be delustered with Titanium Dioxide P 25.
"In the field of dentistry, bulk materials used for the production of false teeth can be tinted with Titanium Dioxide P 25."
This publication does not specify what the "bulk materials used for the production of false teeth" might be made from. False teeth typically are made from porcelain and other ceramics, and from methyl methacrylate and other acrylic resins. If acrylic, these resins are generally polymerized using heat.

Microfine titania is said to be useful for the manufacture of pearlescent automotive paint formulations, as described in U.S. Patent Nos. 4,539,258 and 4,753,829, and in A. Blair Battistini, "Opalescent Colors for Automotive Coatings", American Paint and Coatings Journal, 43-45 (April 8, 1991).

Conventional pigment grade titanium dioxide typically has an average particle size of about 0.2 to 0.5 micrometers. Particles of that size provide optimal scattering of all wave lengths of visible light.

WO-A-90/08799 discloses an improved, castable, synthetic resin matrerial, suitable for use in dental and medical restorations, as a replacement for dental enamel and hydroxyapatite bone material. This material is also useful for electrical insulation. More specifically there is disclosed a filled reinforced resin matrix comprising a castable resin; from 5 to 50% vol of fibers of 50µm or less diameter; from 10 to 35% vol of a first particulate filler having a particle size range of 0.1 to 50µm; and from 5 to 25% vol of a second particulate filler having an ultimate particle size range of 10 to 100nm. As noted on description page 4 line 6 of the reference the second particulate filler may be titania.

US-A-4,141,144 discloses a dental material comprising a polymerizable binder, a polymerization agent and an additive comprising finely divided particles of muscovite mica in a range of from about 1 to 20 percent by weight of the dental material for direct dental filling and restoration applications and in a range of from about 1 to 30 percent for cosmetically treating the surface of a tooth as a veneer or paint-on. Optionally it is disclosed that the muscovite mic can be coated with Tio₂.

### SUMMARY OF THE INVENTION

Several dental manufacturers currently market cold-polymerizable dental restorative materials intended to replicate the optical properties of the dentin or enamel layers of teeth. These products include "DENTACOLOR" restorative from Kulzer, Inc., "CONCEPT" restorative from Vivadent, Inc., "VISIO-GEM" restorative from Espe GmbH, "HERCULITE XR" restorative from the Kerr Division of Sybron, Inc., and "VALUX" and "SILUX PLUS" restoratives from 3M. VALUX restorative contains pigment grade titanium dioxide but no microfine titania. The other commercial materials are not believed to contain microfine titania.

The present invention provides, in one aspect, aesthetic cold-polymerizable dental cements that exhibit lifelike opalescence. In one embodiment this invention provides a cold-polymerizable dental cement comprising:
a) a minor amount of polymerizable resin,
b) a major amount of organic or inorganic filler,
c) a cold-polymerization initiator, and
d) sufficient titania having an average particle diameter less than 0.2 micrometers to visually enhance the opalescence of the cement after the cement has been polymerized, wherein the count of titania is less than 5 weight percent of the cement.

The invention also provides a method for making the above lifelike cold-polmerizable dental cement comprising the step of adding to the material sufficient microfine titania to enhance the opalescence of the material after it has been polymerized.

Other small particle fillers such as submicron silica and submicron alumina can impart a small degree of opalescence to a cold-polymerizable dental material. However, microfine titania appears to be a particularly effective opalizer.

### DETAILED DESCRIPTION

A "cold-polymerizable" dental material is one that contains an initiator that can render the material polymerizable while in the mouth of a patient. The materials of the invention can, if desired, be polymerized outside the mouth (for example, in a dental laboratory).

The dental cements of the invention have "opalescence" when their b* color coordinates exhibit greater yellow levels in transmission (i.e., higher b* color coordinates) or lesser yellow levels in reflection (i.e., lower b* color coordinates) when compared to a material containing no microfine titania, and evaluated using the technique described below in EXAMPLE 1. b* Color coordinates can be obtained by CIELAB (CIE 1978) color determination as described in Billmeyer & Saltzman, Principles of Color Technology, 2nd Ed., pp. 62-65 (1981). Preferably the Δb* value (that is, the transmission b* color coordinate minus the reflection b* color coordinate) for a 1 mm thick sample of the material (for example, the "modified restorative", that is, a restorative containing microfine titania) minus the Δb* value for the unmodified material is greater than 9, more preferably greater than 15, and most preferably greater than 20.

The dental cements of the present invention contain polymerizable resin. The polymerized resin preferably has sufficient strength and hydrolytic stability to render it suitable for use in the mouth. Acceptable polymerizable resins will be familiar to those skilled in the art, and include acrylate, methacrylate, urethane acrylate and urethane methacrylate resins such as those referred to in column 3, lines 13-27 of U.S. Patent No. 4,503,169. A preferred polymerizable resin for use in the present invention is a mixture of diglycidylmethacrylate of bisphenol A (frequently referred to as "Bis-GMA") and triethyleneglycol dimethacrylate (frequently referred to as "TEGDMA").

The dental cements of the present invention also contain organic or inorganic filler. The filler preferably has sufficient strength, translucency (or transparency), color, hydrolytic stability, particle size and shape to render it suitable for use in the mouth. Acceptable fillers will be familiar to those skilled in the art and include silica (e.g., quartz), aluminum oxide, barium oxide, aluminum silicate, lithium aluminum silicate, barium glasses, silicate glasses, fluoroaluminosilicate glasses, phosphate glasses, zinc glasses and zirconia:silica microparticles such as those described in U.S. Patent No. 4,503,169. Suitable organic fillers include ground polymerized acrylate and methacrylate resins. Preferably the ground polymerized resin contains an inorganic filler (e.g., finely-divided amorphous silica) of the type referred to in U.S. Patent No. 4,281,991.

Dental restorative materials of the so-called "hybrid" variety are particularly preferred for use in this invention. These contain fillers whose particles are small in size but not as small as those in the so-called "microfill" dental restoratives. In general, fillers for use in such hybrid dental restoratives have an average particle size between about 0.2 micrometers and about 10 micrometers. They have good physical properties but less than optimum aesthetics. If modified according to the present invention, a hybrid dental restorative can be made much more lifelike in appearance. Microfill dental restoratives (whose fillers typically have an average particle size less than about 0.1 micrometers) can also be rendered more opalescent by adding microfine titania according to the present invention. However, the improvement in opalescence typically is less pronounced than is the case for hybrid dental restoratives.

The polymerizable resin and filler for a cement are present in "minor amount" and "major amount", respectively. By this is meant that the weight percent of the polymerizable resin is less than the weight percent of the filler. Expressed on a weight basis, a preferred resin amount is 10 to 40 percent of the cement, more preferably 15 to 55 percent, more preferably 85 to 67 percent, including the weight of any silane treatment that may be present.

The dental materials of the invention also contain a cold polymerization initiator. Suitable cold polymerization initiators include thermal initiators and photoinitiators. Representative thermal initiators include peroxide compounds alone or in combination with suitable amines, sulfur compounds, phosphorus compounds and other compounds capable of reacting with the peroxide to generate free radicals. Suitable photoinitiators include ketone or alpha diketone compounds alone or in combination with suitable amines, peroxides, sulfur compounds, phosphorus compounds and other compounds capable of reacting with or being sensitized by the ketone or alpha diketone to effect polymerization of the resin.

The amount of polymerization initiator should be sufficient to permit rapid polymerization at room temperature, e.g., in less than 10 minutes, more preferably less than 5 minutes. For polymerization carried out outside the mouth heat may, if desired, be used to accelerate polymerization. Preferably, the total amount of polymerization initiator is less than 5 weight percent of the dental cement, and more preferably 0.1 to 2 weight percent.

Suitable microfine titania is available from a variety of sources. Acceptable materials include "TITANIUM DIOXIDE P 25" from Degussa and "MICRO TITANIUM DIOXIDE" grades MT-100HD, -100S, -100SA, - 100T, -150W, -500B, -500HD, -500SA, -600B and CT-70 from Teikoku Kako Co., Ltd. or from Dainichiseika Color and Chemicals America, Inc. The microfine titania particles have an average particle diameter less than one-half the shortest wave length of visible light, i.e., they have an average particle diameter less than 0.2 micrometers. More preferably, the microfine titania particles have an average particle diameter less than 0.1 micrometers, and more preferably less than 0.06 micrometers. The particles can be made of rutile, anatase or brookite titanium dioxide. The particles can be essentially pure titania, or if desired can contain (or be coated with) or doped with materials such as alumina, silica, zirconia, laurates (e.g., aluminum laurate), stearates (e.g., aluminum stearate), and silanols (e.g., hydrolyzed gammamethacryloxypropyl trimethoxysilane).

In general only very small amounts of microfine titania will be required. If too large an amount is employed then the dental material will become opaque and lose its lifelike appearance.

For a cement, the amount of microfine titania preferably is less than 5 wt. %, preferably 0.01 to 4 wt. % and more preferably 0.05 to 2.5 wt. % of the dental cement.

If desired, the cements of the invention can contain other adjuvents such as polymerization accelerators, inhibitors, stabilizers, pigments, colorants, dyes, viscosity modifiers, extending or reinforcing fillers, surface tension depressants and wetting aids, soluble fluorides, antioxidants, and other ingredients well known to those skilled in the art.

The ingredients of the dental cement can be combined by mixing them in any convenient order using mixing devices of the type typically used in dental manufacturing. Preferably, the microfine titania and filler are dry-blended and then added to a mixture of the polymerizable resin and initiator.

The dental cements of the invention are packaged and dispensed using conventional techniques.

They can be employed for anterior and posterior cement applications, including cementation of inlays and onlays and luting of veneers, crowns, bridges and other prosthetic devices to teeth. As noted above, the materials can be shaped and cured in the mouth or if desired, externally.

The following examples are offered to aid understanding of the present invention and are not to be construed as limiting the invention's scope.

### REFERENCE EXAMPLE 1

25.5 Parts silica sol ("LUDOX" LS, E. I. du Pont de Nemours & Co.) were acidified by the rapid addition of 0.255 parts concentrated nitric acid. In a separate vessel, 12.9 parts ion-exchanged zirconyl acetate (Magnesium Elektron Inc.) were diluted with 20 parts deionized water and the resultant solution acidified with 0.255 parts concentrated nitric acid. The silica sol was pumped into the stirred zirconyl acetate solution and mixed for one hour while filtering the stirred mixture through "CUNO" 5 micrometer and 1 micrometer filters (Commercial Intertech Corp.). The stirred, filtered mixture was further filtered through a 1 micrometer "HYTREX" filter (Osmonics, Inc.) followed by a 0.22 micrometer "BALSTON" filter (Balston Inc.). The filtrate was poured into trays to a depth of about 25 mm and dried at 65°C in a forced air oven for about 24 hours. The resulting dried material was removed from the oven and tumbled through a rotary tube furnace (Harper Furnace Corporation) preheated to 600°C. 21 Parts of calcined microparticles were obtained. The calcined microparticles were comminuted in a tumbling ball mill until all of the microparticles were less than 10 micrometers in particle diameter. 0.3 Part portions of the milled microparticles were placed in ceramic saggers and fired in an electric kiln (Harper Furnace Corporation) in air at 825°C for 1 hour. The fired microparticles were allowed to cool in air. The cooled microparticles were slurried in hydrolyzed gamma-methacryloxypropyl trimethoxysilane, dried in a forced air oven and screened through a 74 micrometer screen. The treated filler particles contained 11.1% silane.

The b* color coordinates for standard daylight conditions are measured for each disk using a "DIANO MATCH SCAN II" color computer (Bausch & Lomb Inc.) with a 25 mm diameter sample port. The b* transmission color coordinates were obtained using the standard white color tile in the reflection sample port. The b* reflection color coordinates were obtained using the standard black color tile behind the cured disk.

### EXAMPLE I

### Preparation of a Dental Cement

Treated filler particles were prepared as detailed in Reference Example 1, except that the milled microparticles were fired at 1010°C for 2 hours and the treated filler particles contained 2.7% silane.

Paste "A" of a two part cold-polymerizable dental cement was prepared by mixing 84.0 parts of the fired, silane-treated microparticles with a resin solution containing 15.7 parts of a 50:50 mixture of Bis-GMA:TEGDMA, 0.1% dimethylaminophenethanol, 0.1 parts diphenyliodonium hexafluorophosphate, 0.03 parts camphorquinone and 0.01 parts hydroxytoluene. The resulting dental cement had a 81.7% filler particle loading level, not counting the silane.

Paste "B" of the cement was prepared by mixing 80.0 parts of the fired, silane-treated microparticles with a resin solution containing 19.7 parts of a 50:50 mixture of Bis-GMA:TEGDMA, 0.2 parts benzoyl peroxide and 0.02 parts hydroxytoluene. Part "B" had a 77.9% filler particle loading level, not counting the silane.

Microfine titania ("MICRO TITANIUM DIOXIDE MT-500HD", Teikoku Kako Co., Ltd.) was hand-kneaded into the "A" paste at addition levels of 0.4 and 2.2 weight %. Each "A" paste was formed into 30 mm in diameter disks of 1 mm and 0.3 mm thickness, cured and b* color coordinates measured as described in EXAMPLE 1.

Set out below in TABLE I are the transmission b* and reflection b* color coordinates for each addition level of microfine titania. The table also shows Δb* values, which are obtained by subtracting the reflection b* from the transmission b*.

**Table I**

| **Disk Thickness** | **b* Color Coordinates % Additive in Paste A** | | |
|---|---|---|---|
| | 0 | 0.4 | 2.2 |
| | | | |

| 1 mm | | | |
|---|---|---|---|
| Transmission | 12.04 | 27.15 | 39.63 |
| Reflection | 4.29 | -2.30 | 0.00 |
| Δb* | 7.75 | 29.45 | 39.63 |

| 0.3 mm | | | |
|---|---|---|---|
| Transmission | 3.33 | 12.32 | 22.27 |
| Reflection | -2.70 | -10.15 | -7.36 |
| Δb* | 6.03 | 22.47 | 29.63 |

The data in TABLE I show that the addition of a small amount of microfine titania to a cold-polymerizable dental cement paste noticeably increases opalescence. The large Δb* values correspond to a visually evident opalescent quality in the cured dental cement. For example, the magnitude of the Δb* values for the 0.4 and 2.2 wt. % addition levels is indicative of a very noticeable improvement in opalescence. Even when the thickness of the disks was reduced by two-thirds (i.e. 0.3 mm), a high degree of opalescence was retained. This is particularly important for veneer applications where only a thin layer of the dental cement paste (luting cement) may be present to contribute to enhancement of the lifelike appearance of the restoration.

One or both pastes can be modified with the addition of microfine titania, pigments, dyes, and viscosity modifiers (i.e., "AEROSIL R-972" from Degussa) to provide pastes with varying pigmentation for adjusting the shade of the final dental restoration cement. For example, the amount of microfine titania, pigments, dyes and viscosity modifiers added to a luting cement can be adjusted by one skilled in the art to provide a desired opalescent quality as well as masking the coloration (i.e., tetracycine staining) or imperfections of the underlying tooth structure. This is particularly important for thin restorations such as veneers.

The "A" paste alone can be used for applications where light curing through a thin section of a composite or porcelain restorative material is desired. It has particular application for final placement of veneers. The "B" paste alone has application for provisional placement of a restorative material such as a veneer to determine shade acceptability. A mixture of paste "A" and paste "B" (preferably a 1:1 weight mixture) can be used for placement of veneers (particularly very thick or opaque veneers), inlays, onlays, crowns, bridges and other dental appliances where limited light curing capability can be utilized.

Although this invention has been described using certain illustrative examples, it should be understood that the invention is not limited to the specific exemplary embodiments shown in this specification.

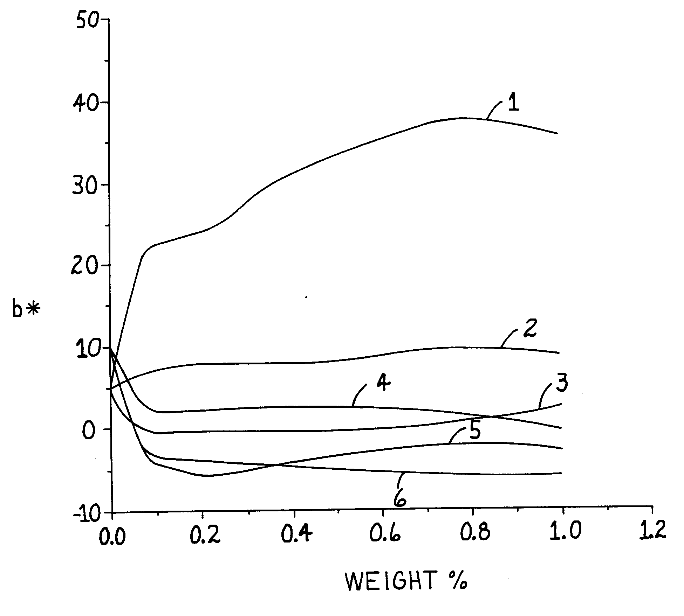

## Claims

1. A cold-polymerizable dental cement comprising:
a) a minor amount of polymerizable resin,
b) a major amount of organic or inorganic filler,
c) a cold-polymerization initiator, and
d) sufficient titania having an average particle diameter less than 0.2 micrometers to visually enhance the opalescence of the cement after the cement has been polymerized, wherein the amount of titania is less than 5 weight percent of the cement.

2. A cement according to claim 1, wherein the transmission b* color coordinate is greater than that of a similar cement containing no titania having an average particle diameter less than 0.2 micrometers.

3. A cement according to claim 1, wherein the reflection b* color coordinate is less than that of a similar cement containing no titania having an average particle diameter less than 0.2 micrometers.

4. A cement according to claim 1, wherein the transmission b* color coordinate is greater and the reflection b* color coordinate is less than that of a similar cement containing no titania having an average particle diameter less than 0.2 micrometers.

5. A cement according to any preceding claim, wherein the Δb* value of the cement minus the Δb* value of a similar cement containing no titania having an average particle diameter less than 0.2 micrometers is greater than 9.

6. A cement according to any preceding claim, wherein the Δb* value of the cement minus the Δb* value of a similar cement containing no titania having an average particle diameter less than 0.2 micrometers is greater than 15.

7. A cement according to any preceding claim, wherein the Δb* value of the cement minus the Δb* value of a similar cement containing no titania having an average particle diameter less than about 0.2 micrometers is greater than 20.

8. A cement according to any preceding claim, wherein the titania particles have an average particle diameter less than 0.1 micrometers.

9. A cement according to any preceding claim, wherein the amount of resin is 10 to 40 weight percent, the amount of filler is 85 to 55 weight percent, and the amount of initiator is less than 5 weight percent.

10. A cement according to any preceding claim, wherein the amount of resin is 15 to 30 weight percent, the amount of filler is 85 to 67 weight percent, and the amount of initiator is 0.1 to 2 weight percent.

11. A cement according to any preceding claim, wherein the amount of titania having an average particle diameter of less than 0.2 micrometers is 0.01 to 4 weight percent of the cement.

12. A cement according to any preceding claim, wherein the amount of titania having an average particle diameter of less than 0.2 micrometers is 0.05 to 2.5 weight percent of the cement.

13. A cement according to any preceding claim useful for cementation of a dental appliance selected from a veneer, inlay, onlay, crown and bridge.

14. A method for making the lifelike cold-polymerizable dental cement of any preceding claim comprising the steps of adding to the polymerizable cement sufficient titania having an average particle diameter of less than 0.2 micrometers to visually enhance the opalescence of the cement after it has been polymerized, wherein the amount of titania having an average particle diameter of less than 0.2 micrometers is less than 5 weight percent of the cement.

15. A method according to claim 14, wherein the amount of titania having an average particle diameter of less than 0.2 micrometers is 0.01 to 4 weight percent of the cement.

16. A method according to claim 15, wherein the amount of titania having an average particle diameter of less than 0.2 micrometers is 0.05 to 2.5 weight percent of the cement.

## Patentansprüche

1. Kaltpolymerisierbarer Dentalzement, umfassend:
(a) eine geringe Menge polymerisierbares Harz;
(b) eine größere Menge eines organischen oder anorganischen Füllmaterials;
(c) einen Initiator für Kaltpolymerisation; sowie
(d) ausreichend Titandioxid mit einem mittleren Partikeldurchmesser von weniger als 0,2 Mikrometer, um die Opaleszenz des Zements sichtbar zu verstärken, nachdem der Zement polymerisiert worden ist, wobei die Menge des Titandioxids weniger als 5 Gewichtsprozent des Zements beträgt.

2. Zement nach Anspruch 1, bei welchem die Transmissions-Farbkoordinate b* größer ist als die eines ähnlichen Zements, der kein Titandioxid mit einem mittleren Partikeldurchmesser von weniger als 0,2 Mikrometer enthält.

3. Zement nach Anspruch 1, bei welchem die Reflexions-Farbkoordinate b* kleiner ist als die eines ähnlichen Zements, der kein Titandioxid mit einem mittleren Partikeldurchmesser von weniger als 0,2 Mikrometer enthält.

4. Zement nach Anspruch 1, bei welchem die Transmissions-Farbkoordinate b* größer und die Reflexions-Farbkoordinate b* kleiner ist als die eines ähnlichen Zements, der kein Titandioxid mit einem mittleren Partikeldurchmesser von weniger als 0,2 Mikrometer enthält.

5. Zement nach einem der vorgenannten Ansprüche, bei welchem der Δb*-wert des Zements minus den Δb*-Wert eines ähnlichen Zements, der kein Titandioxid mit einem mittleren Partikeldurchmesser von weniger als 0,2 Mikrometer enthält, größer ist als 9.

6. Zement nach einem der vorgenannten Ansprüche, bei welchem der Δb*-wert des Zements minus den Δb*-Wert eines ähnlichen Zements, der kein Titandioxid mit einem mittleren Partikeldurchmesser von weniger als 0,2 Mikrometer enthält, größer ist als 15.

7. Zement nach einem der vorgenannten Ansprüche, bei welchem der Δb*-wert des Zements minus den Δb*-Wert eines ähnlichen Zements, der kein Titandioxid mit einem mittleren Partikeldurchmesser von weniger als 0,2 Mikrometer enthält, größer ist als 20.

8. Zement nach einem der vorgenannten Ansprüche, bei welchem die Titandioxid-Partikel einen mittleren Durchmesser von weniger als 0,1 Mikrometer haben.

9. Zement nach einem der vorgenannten Ansprüche, bei welchem die Harzmenge 10 % ... 40 Gewichtsprozent, die Füllstoffmenge 85 % ... 55 Gewichtsprozent und die Menge des Initiators weniger als 5 Gewichtsprozent beträgt.

10. Zement nach einem der vorgenannten Ansprüche, bei welchem die Harzmenge 15 % ... 30 Gewichtsprozent, die Füllstoffmenge 85 % ... 67 Gewichtsprozent und die Menge des Initiators 0,1 % ... 2 Gewichtsprozent beträgt.

11. Zement nach einem der vorgenannten Ansprüche, bei welchem die Menge Titandioxid, das einen mittleren Partikeldurchmesser von weniger als 0,2 Mikrometer hat, 0,01 % ... 4 Gewichtsprozent des Zements beträgt.

12. Zement nach einem der vorgenannten Ansprüche, bei welchem die Menge Titandioxid, das einen mittleren Partikeldurchmesser von weniger als 0,2 Mikrometer hat, 0,05 % ... 2,5 Gewichtsprozent des Zements beträgt.

13. Zement nach einem der vorgenannten Ansprüche, verwendbar zur Zementierung eines Dentalapparates, ausgewählt aus einem Auflagewerkstoff, Inlay, Onlay, Krone und Brücke.

14. Verfahren zum Herstellen des lebensnahen, kaltpolymerisierbaren Dentalzements nach einem der vorgenannten Ansprüche, umfassend die Schritte des Zusetzens von ausreichend Titandioxid mit einem mittleren Partikeldurchmesser von weniger als 0,2 Mikrometer, um die Opaleszenz des Zements sichtbar zu verstärken, nachdem der Zement polymerisiert worden ist, wobei die Menge des Titandioxids mit einem mittleren Partikeldurchmesser von weniger als 0,2 Mikrometer weniger als 5 Gewichtsprozent des Zements beträgt.

15. Verfahren nach Anspruch 14, bei welchem die Menge des Titandioxids, das einen mittleren Partikeldurchmesser von weniger als 0,2 Mikrometer hat, 0,01 % ... 4 Gewichtsprozent des Zements beträgt.

16. Verfahren nach Anspruch 15, bei welchem die Menge des Titandioxids, das einen mittleren Partikeldurchmesser von weniger als 0,2 Mikrometer hat, 0,05 % ... 2,5 Gewichtsprozent des Zements beträgt.

## Revendications

1. Ciment dentaire polymérisable à froid comprenant :
a) une quantité mineure de résine polymérisable,
b) une quantité majeure d'une charge organique ou inorganique,
c) un initiateur de polymérisation à froid, et
d) une quantité suffisante d'oxyde de titane ayant un diamètre particulaire moyen inférieur à 0,2 µm pour améliorer visuellement l'opalescence du ciment après que le ciment ait été polymérisé, la quantité de titane étant inférieure à 5 % en produit ciment.

2. Ciment selon la revendication 1, dans lequel la coordonnée de couleur en transmission b* est supérieure à celle d'un ciment similaire ne contenant pas d'oxyde de titane ayant un diamètre particulaire moyen inférieur à 0,2 µm.

3. Ciment selon la revendication 1 dans lequel la coordonnée de couleur en réflexion b* est inférieure à celle d'un ciment similaire ne contenant pas d'oxyde de titane et ayant un diamètre particulaire moyen inférieur à 0,2 µm.

4. Ciment selon la revendication 1, dans lequel la coordonnée de couleur en transmission b* est supérieure et la coordonnée de couleur b* en transmission est inférieure à celle d'un ciment similaire ne contenant pas d'oxyde de titane et ayant un diamètre particulaire moyen inférieur à 0,2 µm.

5. Ciment selon l'une quelconque des revendications précédentes, dans lequel la valeur de Δb* du ciment diminuée de la valeur de Δb* d'un ciment similaire ne contenant pas d'oxyde de titane ayant un diamètre particulaire moyen inférieur à 0,2 µm est supérieure à 9.

6. Ciment selon l'une quelconque des revendications précédentes, dans lequel la valeur de Δb* du ciment diminuée de la valeur de Δb* d'un ciment similaire ne contenant pas d'oxyde de titane et ayant un diamètre particulaire moyen inférieur à 0,2 µm est supérieure à 15.

7. Ciment selon l'une quelconque des revendications précédentes, dans lequel la valeur de Δb* du ciment diminuée de la valeur de Δb* d'un ciment similaire ne contenant pas d'oxyde de titane et ayant un diamètre particulaire moyen inférieur à environ 0,2 µm est supérieure à 20.

8. Ciment selon l'une quelconques des revendications précédentes, dans lequel lesdites particules d'oxyde de titane ont un diamètre particulaire moyen inférieur à 0,1 µm.

9. Ciment selon l'une quelconque des revendications précédentes, dans lequel la quantité de résine est de 10 à 40 % en poids, la quantité de charge est de 85 à 55 % en poids, et la quantité d'initiateur est inférieure à 5 % en poids.

10. Ciment selon l'une quelconque des revendications précédentes, dans lequel la quantité de résine est de 15 à 30 % en poids, la quantité de charge est de 85 à 67 % en poids, et la quantité d'initiateur est de 0,1 % à 2 % en poids.

11. Ciment selon l'une quelconque des revendications précédentes, dans lequel la quantité d'oxyde de titane ayant un diamètre particulaire moyen inférieur à 0,2 µm est de 0,01 à 4 % en poids du ciment.

12. Ciment selon l'une quelconque des revendications précédentes, dans lequel la quantité d'oxyde de titane ayant un diamètre particulaire moyen inférieur à 0,2 µm est de 0,05 à 2,5 % en poids du ciment.

13. Ciment selon l'une quelconque des revendications précédentes utile pour la cimentation d'un appareil dentaire choisi parmi un placage, une incrustation, une application, une couronne et un bridge.

14. Procédé de fabrication du ciment dentaire polymérisable à froid semblable au matériau vivant de l'une quelconque des revendications précédentes comprenant les étapes d'addition au ciment polymérisable d'une quantité suffisante d'oxyde de titane ayant un diamètre particulaire moyen inférieur à 0,2 µm pour améliorer à l'oeil nu l'opalescence du ciment après qu'il ait été polymérisé, où la quantité d'oxyde de titane ayant un diamètre particulaire moyen inférieur à 0,2 µm est inférieure à 5 % en poids du ciment.

15. Procédé selon la revendication 14, dans lequel la quantité d'oxyde de titane ayant un diamètre particulaire moyen inférieur à 0,2 µm est de 0,01 à 4 % en poids du ciment.

16. Procédé selon la revendication 15, dans lequel la quantité d'oxyde de titane ayant un diamètre particulaire moyen inférieur à 0,2 µm est de 0,05 à 2,5 % en poids du ciment.
